(19) Europäisches Patentamt / European Patent Office / Office européen des brevets

(11) **EP 3 995 129 B1**

(12) **EUROPEAN PATENT SPECIFICATION**

(45) Date of publication and mention of the grant of the patent:
**29.03.2023 Bulletin 2023/13**

(21) Application number: **21190536.9**

(22) Date of filing: **10.08.2021**

(51) International Patent Classification (IPC):
*A61K 8/9789* (2017.01)    *A61Q 19/00* (2006.01)
*A61Q 19/08* (2006.01)

(52) Cooperative Patent Classification (CPC):
**A61K 8/9789; A61Q 19/00; A61Q 19/007; A61Q 19/08;** A61K 2800/85

(54) **COSMETIC COMPOSITION COMPRISING DAHLIA PINNATA FERMENTED EXTRACT AS ACTIVE INGREDIENT, DAHLIA PINNATA COMPOSITION AND METHOD FOR PREPARING THE SAME**

KOSMETISCHE ZUSAMMENSETZUNG MIT FERMENTIERTEM DAHLIA-PINNATA-EXTRAKT ALS WIRKSTOFF, DAHLIA-PINNATA-ZUSAMMENSETZUNG UND VERFAHREN ZUR HERSTELLUNG DAVON

COMPOSITION COSMÉTIQUE COMPRENANT UN EXTRAIT DE DAHLIA PINNATA FERMENTÉ EN TANT QU'INGRÉDIENT ACTIF, COMPOSITION DE DAHLIA PINNATA ET SON PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.11.2020 KR 20200148277**
**30.04.2021 KR 20210056750**

(43) Date of publication of application:
**11.05.2022 Bulletin 2022/19**

(73) Proprietor: **Baram International Co., Ltd.**
**Seoul 06021 (KR)**

(72) Inventors:
- **PARK, Rae Hyun**
  **06592 Seoul (KR)**
- **LEE, Sang Jae**
  **46957 Busan (KR)**
- **PARK, Mi Hwa**
  **46957 Busan (KR)**
- **CHOI, Bo Gyoung**
  **52140 Gyeongsangnam-do (KR)**
- **YEOM, YuJeong**
  **47778 Busan (KR)**
- **GANBAT, Dariimaa**
  **46958 Busan (KR)**
- **JEONG, Ga Eul**
  **46638 Busan (KR)**

- **KIM, Sung Tae**
  **05678 Seoul (KR)**
- **LIM, Hana**
  **46917 Busan (KR)**
- **LEE, Siyeong**
  **50818 Gyeongsangnam-do (KR)**
- **KIM, Eunji**
  **51608 Gyeongsangnam-do (KR)**
- **JEONG, Da-young**
  **50817 Gyeongsangnam-do (KR)**
- **KIM, So Yun**
  **50814 Gyeongsangnam-do (KR)**

(74) Representative: **Prüfer & Partner mbB**
**Patentanwälte · Rechtsanwälte**
**Sohnckestraße 12**
**81479 München (DE)**

(56) References cited:
**WO-A1-2019/146889**

- **ESTRELLA LARA-CORTÉS ET AL: "ANTIOXIDANT CAPACITY, NUTRITIONAL AND FUNCTIONAL COMPOSITION OF EDIBLE DAHLIA FLOWERS", REVISTA CHAPINGO / SERIE HORTICULTURA, vol. XX, no. 1, 1 April 2014 (2014-04-01), pages 101-116, XP055746190, ISSN: 1027-152X, DOI: 10.5154/r.rchsh.2013.07.024**

**Description**

[Technical Field]

The present invention relates to a cosmetic composition comprising Dahlia pinnata fermented extract as an active ingredient, a Dahlia pinnata composition, and a method for preparing the same.

[Background Art]

The skin consists of three layers: the epidermis, the dermis, and the subcutaneous fat, and among them, the epidermis, the outermost layer, is the part of the body that is always in contact with the external environment and acts as a protective barrier. The stratum corneum of the epidermis is composed of a protein composed of dead keratinocytes and lipids present between keratinocytes, and the skin barrier present in this stratum corneum plays an important role in functionally regulating the division and differentiation of keratinocytes to protect the skin, preventing the outflow of substances in the body, and inhibiting the loss of moisture and electrolytes. Therefore, minimizing the lack of moisture and maintaining skin homeostasis by strengthening the skin barrier may be very important for skin protection.

On the other hand, when the skin is excessively exposed to external factors or irritants, skin irritations such as erythema, edema and itching, and immune-inflammatory reactions are induced. It is known that skin troubles due to the skin irritation stress not only cause a problem in appearance, but also affect overall skin health, from skin pigmentation to increase in wrinkles due to the immune-inflammatory reaction process, by-products, etc. (Welss T, Basketter DA, Schröder KR. In vitro skin irritation: fact and future. State of the art review of mechanisms and models. Toxicol In Vitro. 2004;18:231-43). Accordingly, it is desired to develop a composition capable of reducing skin irritation and maintaining skin homeostasis by alleviating immune inflammation.

Meanwhile, Dahlia pinnata is a dicotyledonous perennial plant of the family Chrysanthemum of the order Asteraceae. This Dahlia pinnata is a plant with tuber and herbaceous characteristics, which is native to Mexico and widely cultivated in each country for ornamental purposes. Dahlia pinnata reproduces with roots that look like sweet potatoes. The stem of Dahlia pinnata is cylindrical, well branched, hairless, greenish, and 1.5 to 2m high. The leaves are in opposing pairs and divided into pinnate 1 to 2 times. The small leaves of Dahlia pinnata are egg-shaped, have serrated edges, are dark green on the surface and white on the back, and have some wings on the petiole.

The tuberous root of Dahlia pinnata has an analgesic effect and is used medicinally for toothache and parotitis caused by tooth decay. In addition, Dahlia pinnata petals are effective in diseases such as cardiovascular disease, dementia, and cancer. When such a composition of a Dahlia pinnata flower extracted from Dahlia pinnata petals is used as a raw material for food, it may be used medicinally for diseases such as cardiovascular disease, dementia, and cancer.

It has been reported in International Patent Application No. PCT/KR2018/013754 (WO2019/146889) that the extract of Dahlia pinnata has an antioxidant efficacy, but the inventors of the present application have completed the present invention by founding out that antioxidant and anti-aging efficacy is more significantly improved, and antibacterial, skin barrier strengthening, skin immunity enhancement and inflammation relief are represented, when the extract of Dahlia pinnata is fermented by using one or more Bacillus sp. strains selected from the group consisting of *Bacillus licheniformis* and *Bacillus amyloliquefaciens.*

[Disclosure]

[Technical Problem]

An object of the present invention is to provide a cosmetic composition with significantly improved antioxidant and anti-aging efficacies, in addition to exhibiting the efficacy of antibacterial, skin barrier strengthening, skin immunity enhancement, and inflammation relief.

Another object of the present invention is to provide a Dahlia pinnata composition that may be added to food or cosmetic and may impart antibacterial, skin barrier strengthening, skin immunity enhancement, and inflammation relief effects to food or cosmetics, and significantly excellent antioxidant and anti-aging efficacies; and a method for preparing the composition.

[Technical Solution]

In an aspect, there is provided a cosmetic composition comprising Dahlia pinnata fermented extract as an active ingredient.

In the present invention, the Dahlia pinnata fermented extract may be obtained by inoculating Dahlia pinnata extract with one or more Bacillus sp. strains selected from the group consisting of *Bacillus licheniformis* and *Bacillus*

*amyloliquefacienso*, and then fermenting the extract.

In the present invention, it is preferable that the Bacillus sp. strains are one or more selected from the group consisting of *Bacillus licheniformis* R450403 (Deposit accesion number: KCTC14348BP) and *Bacillus amyloliquefaciens* CTC 2-5-1 (Deposit accession number: KCTC14347BP).

In the present invention, the Dahlia pinnata extract may be an ethanol extract of Dahlia pinnata petals.

In the present invention, the concentration of the Dahlia pinnata fermented extract may be 1 to 20 mg/ml.

In the present invention, a content of total phenolic compounds may be 50 to 80% by weight based on the Dahlia pinnata fermented extract.

In the present invention, the Dahlia pinnata fermented extract may be improved in one or more of 2,2-diphenyl-1-picrylhydrazyl (DPPH) free radical scavenging activity and 2,2'-azino-bis-3-ethyl benzothiazoline-6-sulphonic acid (ABTS) free radical scavenging, compared to the Dahlia pinnata extract.

In the present invention, the cosmetic composition may have antioxidant and anti-aging activities.

In the present invention, the cosmetic composition may be a cosmetic composition for strengthening the skin barrier.

In the cosmetic composition for strengthening the skin barrier, it is preferred that the concentration of the Dahlia pinnata fermented extract is 1 to 10mg/ml.

The cosmetic composition for strengthening the skin barrier may increase an expression of one or more proteins selected from filaggrin and keratin 1 in the skin cell to strengthen the skin barrier.

In the present invention, the cosmetic composition may be an anti-inflammatory cosmetic composition.

In the anti-inflammatory cosmetic composition, it is preferred that the concentration of the Dahlia pinnata fermented extract is 1 to 20mg/ml.

The anti-inflammatory cosmetic composition may represent an anti-inflammatory effect by inhibiting a production of one or more inflammatory factors selected from nitric oxide (NO) and interleukin-1β (IL-1β).

In another aspect, a method for preparing Dahlia pinnata composition comprises the steps of: preparing a Dahlia pinnata extract, inoculating the Dahlia pinnata extract with one or more Bacillus sp. strains selected from the group consisting of *Bacillus licheniformis* and *Bacillus amyloliquefaciens*, and then fermenting the extract to obtain Dahlia pinnata fermented extract, and diluting the Dahlia pinnata fermented extract in a solvent to obtain a Dahlia pinnata composition having antioxidant efficacy.

In the present invention, the fermentation may be performed for 72 to 144 hours at a temperature of 25 to 45°C, preferably at a temperature of 37°C.

In the present invention, the Bacillus sp. strains may be inoculated in an amount of 1 to 10 parts by volume based on 100 parts by volume of the Dahlia pinnata extract.

In the present invention, the step of preparing the Dahlia pinnata extract may include preparing the Dahlia pinnata petals, adding ethanol to the Dahlia pinnata petals and stirring them to extract the Dahlia pinnata extract, and filtering the Dahlia pinnata extract.

In the present invention, the step of extracting the Dahlia pinnata extract may include adding 5 to 20ml of ethanol per 1g of the Dahila pinnata petals to extract the Dahlia pinnata extract.

In the present invention, the step of filtering the Dahlia pinnata extract may include filtering the Dahlia pinnata extract using a syringe filter.

In the present invention, the method may further include freeze-drying the Dahlia pinnata composition, and preparing the freeze-dried Dahlia pinnata composition in a powder form.

In another aspect, there is provided a Dahlia pinnata composition prepared by method as described above and comprising Dahlia pinnata fermented extract.

[Advantageous Effects]

The Dahlia pinnata composition according to the present invention provides Dahlia pinnata fermented extract using a strain capable of fermenting the Dahlia pinnata extract, which cannot be fermented with a normal strain, so that it can be added to food or cosmetics to confer remarkably excellent antioxidant and anti-aging efficacies. In addition, it is possible to exhibit skin barrier strengthening and anti-inflammatory effects by increasing an expression level of the skin keratinocyte protein and moisturizing factors and suppressing generation of inflammatory factors.

[Brief Description of the Drawings]

FIG. 1 is a flowchart of a method for preparing a Dahlia pinnata composition according to an embodiment of the present invention.

FIG. 2 is a flowchart for explaining the step of preparing Dahlia pinnata extract according to an embodiment of the present invention.

FIG. 3 is a flowchart of a method for preparing a Dahlia pinnata composition in a powder form, according to an embodiment of the present invention.

FIG. 4A is a growth curve in *Bacillus licheniformis* R450403, and FIG. 4B is a growth curve in *Bacillus amyloliquefaciens* CTC 2-5-1.

FIG. 5 is a graph showing DPPH radical scavenging ability of Dahlia pinnata extract according to an embodiment of the present invention.

FIG. 6 is a graph showing ABTS radical scavenging ability of Dahlia pinnata extract according to an embodiment of the present invention.

FIG. 7 is a graph showing relative radical scavenging ability of Dahlia pinnata extract according to an embodiment of the present invention.

FIGS. 8A and 8B are graphs showing filaggrin expression level in Dahlia pinnata fermented extract which is fermented with R450403 and CTC 2-5-1 according to an embodiment of the present invention.

FIGS. 9A and 9B are graphs showing keratin 1 expression level in Dahlia pinnata fermented extract which is fermented with R450403 and CTC 2-5-1 according to an embodiment of the present invention.

FIGS. 10A and 10B are graphs showing level of producing the nitrogen monoxide in Dahlia pinnata fermented extract which is fermented with R450403 and CTC 2-5-1 according to an embodiment of the present invention.

FIGS. 11A and 11B are graphs showing level of producing interleukin-1 beta in Dahlia pinnata fermented extract which is fermented with R450403 and CTC 2-5-1 according to an embodiment of the present invention.

[Best Mode]

Unless defined otherwise, all technical and scientific terms used herein have the same meaning as commonly understood by one of ordinary skill in the art to which this invention belongs. In general, the nomenclature used herein is those well known and commonly used in the art.

Throughout the specification, when any part "comprises" any certain component, it does not exclude other components unless otherwise stated, but may further include other components.

The present invention relates to a cosmetic composition comprising Dahlia pinnata fermented extract as an active ingredient.

In the present disclosure, "Dahlia pinnata fermented extract" refers to a product obtained by inoculating a strain for fermentation to Dahlia pinnata extract and fermenting it, and "Dahlia pinnata extract" means a product extracted by adding an extraction solvent such as ethanol to Dahlia pinnata petals.

In the present disclosure, the Dahlia pinnata fermented extract may be a product obtained by inoculating a Bacillus sp. strain to Dahlia pinnata extract and fermenting it.

In the present disclosure, "Dahlia pinnata composition" refers to a solution obtained by diluting Dahlia pinnata fermented extract in a solvent.

In the present disclosure, the concentration of Dahlia pinnata fermented extract refers to the value of mg of Dahlia pinnata fermented extract dissolved in 1ml of the Dahlia pinnata composition.

In the present disclosure, a "cosmetic composition" refers to a cosmetic comprising the Dahlia pinnata fermented extract, and a cosmetic refers to an additive or a combination of additives for preparing cosmetics that does not comprise Dahlia pinnata extract.

Dahlia pinnata petals contain phenolic compounds (polyphenols) that inhibit oxidative damage caused by active oxygen and an aging process thereby, and thus Dahlia pinnata composition comprising Dahlia pinnata fermented extract has antioxidant efficacy, thereby having the effect of inhibiting the oxidative damage and the aging process. As such, the Dahlia pinnata composition has an antioxidant efficacy, and thus may be provided as food or cosmetics.

Meanwhile, Dahlia pinnata petals have a variety of colors depending on the type. Accordingly, the Dahlia pinnata composition may have various colors, and may produce food or cosmetics having various colors.

The present invention provides a cosmetic composition comprising Dahlia pinnata fermented extract obtained by inoculating one or more Bacillus sp. strains selected from the group consisting of *Bacillus licheniformis* and *Bacillus amyloliquefaciens* to the Dahlia pinnata extract and fermenting the extract.

The present inventors have conducted a study on the fermentation of Dahlia pinnata extract for various strains in order to obtain more improved skin improvement activity by fermenting the Dahlia pinnata extract, but found that most of the strains did not grow normally in the Dahlia pinnata extract medium and fermentation did not proceed. Accordingly, the present inventors primarily selected about 400 candidate strains suitable for fermentation of Dahlia pinnata extract from a library of about 1,500 strains, and selected 71 strains with good activity of amylase and protease, etc., suitable for Dahlia pinnata extract with high ratio of protein and sugar, and proceeded with additional experiments on 36 strains of the genus Bacillus that showed growth potential in Dahlia pinnata medium. As a result, it was confirmed that only *Bacillus licheniformis* and *Bacillus amyloliquefaciens* showed a normal growth curve in Dahlia pinnata medium, and only the two strains may produce Dahlia pinnata fermented extract.

Therefore, the challenge of the present invention is to provide a cosmetic composition comprising Dahlia pinnata fermented extract as an active ingredient, obtained by inoculating one or more Bacillus sp. strains selected from the group consisting of *Bacillus licheniformis* and *Bacillus amyloliquefaciens* to Dahlia pinnata extract and fermenting the extract, and it was found that, when at least one Bacillus sp. strain selected from the group consisting of *Bacillus licheniformis* and *Bacillus amyloliquefaciens* is inoculated to Dahlia pinnata extract and the extract is fermented, the effects of strengthening the skin barrier, enhancing skin immunity and alleviating inflammation are exerted, and further the antioxidant and anti-aging efficacies may be significantly improved.

Preferably, Bacillus sp. strains applicable to the present invention may be one or more selected from the group consisting of *Bacillus licheniformis* R450403 and *Bacillus amyloliquefaciens* CTC 2-5-1. Among them, *Bacillus licheniformis* R450403 has been deposited with the Korea Research Institute of Bioscience and Biotechnology as Deposit number KCTC14348BP (Deposit date: October 28, 2020), and *Bacillus amyloliquefaciens* CTC 2-5-1 has been deposited with the Korea Research Institute of Bioscience and Biotechnology as Deposit number KCTC14347BP (Deposit date: October 28, 2020).

As an example, the content of the total phenolic compound may be 50 to 80% by weight, preferably 55 to 75% by weight, and more preferably 60 to 70% by weight, based on Dahlia pinnata fermented extract. This corresponds to more than twice of high level of Dahlia pinnata extract (non-fermented).

As an example, the Dahlia pinnata fermented extract may be extract with at least one of 2,2-diphenyl-1-picrylhydrazyl (DPPH) free radical scavenging activity and 2,2'-azino-bis-3-ethyl benzothiazoline-6-sulphonic acid (ABTS) free radical scavenging activity improved, compared to the Dahlia pinnata extract (non-fermented). As a preferred example, the Dahlia pinnata fermented extract may improve both DPPH free radical scavenging activity and ABTS free radical scavenging activity by 1.4 times or more, compared to Dahlia pinnata extract (non-fermented).

Accordingly, the cosmetic composition of the present invention may exhibit antioxidant and anti-aging activities by comprising the Dahlia pinnata fermented extract.

In the cosmetic composition exhibiting the antioxidant and anti-aging activities, the concentration of the Dahlia pinnata fermented extract may be 1 to 20mg/ml. Specifically, the concentration of the Dahlia pinnata fermented extract which is fermented with *Bacillus licheniformis* is preferably 3.45 to 14.25mg/ml, more preferably 7.125 to 14.25mg/ml, and the concentration of the Dahlia pinnata fermented extract which is fermented with *Bacillus amyloliquefaciens* is preferably 3.38 to 13.5mg/ml, and more preferably 6.75 to 13.5mg/ml.

The cosmetic composition of the present invention may strengthen the skin barrier by comprising the Dahlia pinneta fermented extract.

The epidermis, located at the outermost part of the skin, performs a protective function against external stimuli and prevents excessive evacuation of body moisture, and for this function, the stratum corneum composed of keratinocytes needs to be normally formed and maintained. Keratinocytes form a stratum corneum while producing natural moisturizing factor (NMF) and intercellular lipids, thereby acting as a physical barrier and permeation barrier in the skin.

This skin barrier may be strengthened by enhancing the aggregation of proteins forming the stratum corneum and increasing the moisturizing factor of the skin. Specifically, the protein forming the stratum corneum may strengthen the physical barrier of the skin by forming keratinocytes with a hard and flat structure, and moisturizing factors excellent in absorbing moisture act as a humectant in the stratum corneum and help enzymes of the skin to function normally.

In the present invention, the Dahlia pinnata fermented extract may strengthen the skin barrier by increasing the expression levels of proteins and moisturizing factors in the formation of the stratum corneum of the skin. Specifically, in the examples of the present invention, it was confirmed that the expression levels of filaggrin and keratin 1 were increased when the keratinocytes were treated with the Dahlia pinnata composition comprising Dahlia pinnata fermented extract.

Keratin is a protein synthesized as keratinocytes differentiate into corneocytes, and accounts for more than 50% of stratum corneum cells and about 80% of the total protein in the stratum corneum. Since keratin fibers are distributed throughout the stratum corneum cells and serve to form a physical barrier, when the expression level of keratin is increased, a hard barrier may be formed on the skin to protect the skin from external stimuli. Among keratin proteins, examples of normal epidermal structural proteins include keratin 1, keratin 10, etc.

Filaggrin is a keratin-binding protein and functions to strengthen the barrier by promoting the binding between keratin microfibers. Thereafter, filaggrin is decomposed into amino acids as it rises to the upper part of the stratum corneum, and free amino acids and pyrrolidone carboxylic acid (PCA), which are natural moisturizing factors, are generated. Thus, filaggrin not only strengthens the skin barrier, but also plays very important role in keeping the skin moist by its decomposition products. In addition, filaggrin produces citrulline, PCA, and trans-UCA through the decomposition process, thereby helping to hydrate the stratum corneum, normalize pH, form a permeation barrier, and have antibacterial and anti-inflammatory effects. Therefore, filaggrin not only strengthens the skin barrier and acts as a natural moisturizing factor, but is also related to the improvement of dermatitis.

Dahlia pinnata fermented extract of the present invention may increase the expression of filaggrin and keratin 1, thereby strengthening the physical barrier of the skin to protect the skin from external stimuli, and exhibiting moisturizing effects.

In the cosmetic composition for strengthening the skin barrier according to the present invention, the higher the concentration of the Dahlia pinnata fermented extract, the higher the effect of strengthening skin barrier may be. For example, the concentration of the Dahlia pinnata extract may be 1 to 20mg/ml, and preferably 1 to 10mg/ml. Specifically, the concentration of the Dahlia pinnata fermented extract which is fermented with *Bacillus licheniformis* may be 3 to 8mg/ml, and preferably 4 to 6mg/ml, and the concentration of the Dahlia pinnata fermented extract which is fermented with *Bacillus amyloliquefaciens* may be 2 to 8mg/ml, and preferably 4 to 6mg/ml.

In addition, the cosmetic composition of the present invention may exhibit anti-inflammatory effect by comprising the Dahlia pinnata fermented extract.

In the present disclosure, the term "anti-inflammatory" refers to the activity of alleviating or inhibiting inflammation. Inflammation is a defense mechanism of living tissues against various stimuli from inside or outside the human body, and activated macrophages secrete inflammation-inducing cytokines and excessively produce inflammatory mediators to further activate the inflammatory process.

Examples of the inflammatory cytokines include interleukin-1 beta (interleukin-1$\beta$, IL-1$\beta$), interleukin-6 (IL-6), tumor necrosis factor-$\alpha$ (TNF-$\alpha$), etc. For example, IL-1$\beta$ activates macrophages, promotes adhesion of lymphocytes and neutrophils to endothelial cells, and increases the infiltration of inflammatory cells into inflammatory sites. TNF-$\alpha$ induces neutrophils to the site in the early stage of the inflammatory response, and is a factor that causes and exacerbates the acute inflammatory response. In addition, as a representative inflammatory cytokine, IL-6 is synthesized and secreted by various factors and is involved in acute or chronic inflammatory diseases.

Representative examples of inflammatory mediators include nitric oxide (NO) and prostaglandin E2 (PGE2). It is known that overexpressed NO in the inflammatory response increases vascular permeability, causing edema and intensifying inflammation, which induces various cell and tissue damage and results in chronic inflammatory diseases and autoimmune diseases. Like NO, PGE2 is also an important inflammatory mediator mainly involved in the transmission of pain and fever in damaged areas or tissues, and when produced in excess, causes an excessive immune response to induce various inflammatory diseases.

The Dahlia pinnata fermented extract according to the present invention may exhibit anti-inflammatory effects by inhibiting the production and expression of inflammatory factors such as these inflammatory cytokines and inflammatory mediators. Specifically, in the examples of the present invention, it was confirmed that the concentrations of IL-1$\beta$ and NO were reduced when the cells induced in inflammation were treated with a composition comprising the Dahlia pinnata fermented extract.

Accordingly, the anti-inflammatory cosmetic composition according to the present invention comprising the Dahlia pinnata fermented extract as an active ingredient may have an effect of improving skin diseases related to inflammation. For example, through the anti-inflammatory effect of the present invention, the effect of improving atopic dermatitis, psoriasis, erythematous disease triggered by radiation, chemicals, burns, etc., itching due to allergy, inflammatory disease such as seborrheic eczema, or skin troubles such as acne may be exhibited.

In the anti-inflammatory cosmetic composition according to the present invention, the higher the concentration of the Dahlia pinnata fermented extract is, the higher the effect for anti-inflammatory response may be. For example, the concentration of Dahlia pinnata fermented extract may be 1 to 20mg/ml. Specifically, the concentration of the Dahlia pinnata fermented extract which is fermented with *Bacillus licheniformis* may be 4 to 15mg/ml, and preferably 6 to 10mg/ml, and the concentration of the Dahlia pinnata fermented extract which is fermented with *Bacillus amyloliquefaciens* may be 4 to 15mg/ml, and preferably 8 to 12mg/ml.

Accordingly, Dahlia pinnata fermented extract is added to the various cosmetics such as face wash cosmetics, basic cosmetics, color cosmetics, hair cosmetics, functional cosmetics, etc. to confer the functionality of showing antioxidant, skin barrier strengthening and anti-inflammatory activity to cosmetics. In addition, since the cosmetic composition comprising the Dahlia pinnata fermented extract has various colors depending on the color of Dahlia pinnata petals as raw materials, it may be used as a natural pigment for color cosmetics such as makeup base, foundation, lipstick, eye shadow, nail polish, etc.

The present invention also provides a method for preparing Dahlia pinnata composition, and a Dahlia pinnata composition prepared by the method.

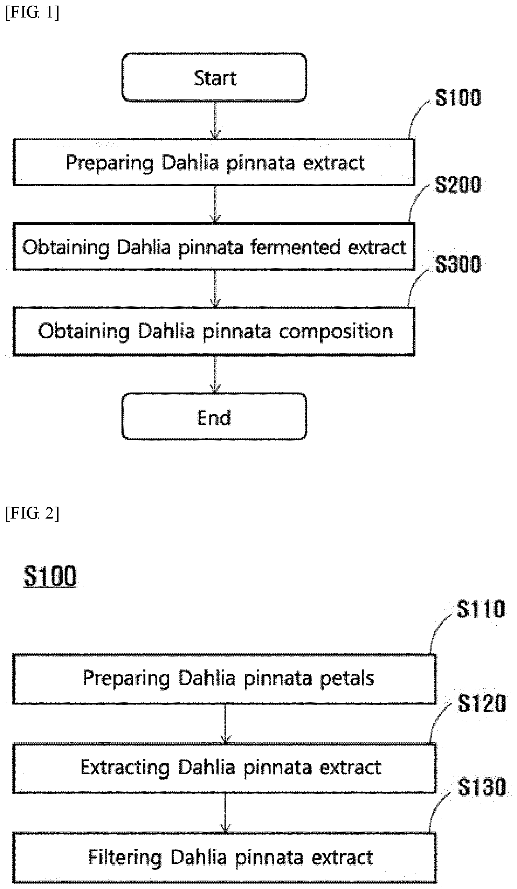

FIG. 1 is a flowchart of a method for preparing a Dahlia pinnata composition according to an embodiment of the present invention.

Referring to FIG. 1, a method for preparing a Dahlia pinnata composition may comprise the steps of: preparing Dahlia pinnata extract (S 100), inoculating the Dahlia pinnata extract with Bacillus sp. strains selected from the group consisting of *Bacillus licheniformis* and *Bacillus amyloliquefaciens*, and then fermenting the extract to obtain Dahlia pinnata fermented extract (S200), and diluting the Dahlia pinnata fermented extract in a solvent to obtain a Dahlia pinnata composition (S300).

In the step of obtaining the Dahlia pinnata fermented extract (S200), it may be fermented after inoculating the Dahlia pinnata extract with one or more Bacillus sp. strains selected from the group consisting of *Bacillus licheniformis* and *Bacillus amyloliquefaciens.*

Fermentation refers to a process in which enzymes possessed by microorganisms change raw materials to create beneficial ingredients, and during the fermentation process, active ingredients are extracted and the content of functional substances increases. The present inventors have found that when fermenting Dahlia pinnata extract using one or more Bacillus sp. strains selected from the group consisting of *Bacillus licheniformis* and *Bacillus amyloliquefaciens*, the content of phenolic compounds was significantly increased, the protein expression level to strengthen the skin barrier was increased, and the concentration of inflammatory factors was decreased.

As an example, the fermentation may be performed at a temperature of 25 to 45°C, preferably at a temperature of 37°C for 72 to 144 hours.

As an example, the Bacillus sp. strains may be inoculated in an amount of 1 to 10 parts by volume based on 100 parts by volume of the Dahlia pinnata extract.

In the step of obtaining the Dahlia pinnata composition (S300), the Dahlia pinnata fermented extract is diluted in a solvent to adjust the concentration of Dahlia pinnata extract to prepare a Dahlia pinnata composition. As an example, ethanol may be used as the solvent, but is not necessarily limited thereto.

Such a Dahlia pinnata composition may be ingested by adding it to food in liquid form, and may also be possible to add it to cosmetics. In other words, the Dahlia pinnata composition is composed of ethanol wherein the Dahlia pinnata extract is diluted, and it may be possible to prepare a cosmetic composition by adding the Dahlia pinnata composition to cosmetics or food.

As another example, the solvent may be a cosmetic. In this case, it is possible to prepare a cosmetic composition including Dahlia pinnata fermented extract by adding the Dahlia pinnata fermented extract to the cosmetic.

As an example, the concentration of Dahlia pinnata fermented extract in the cosmetic composition may be 1 to 20mg/ml. In this case, effects such as antioxidant, whitening, anti-aging, wrinkle improvement, antibacterial, skin barrier strengthening, skin immunity enhancement, and inflammation relief may be maximized.

Herein, the cosmetic composition may include cosmetics comprising various additives of different ingredients depending on the type of cosmetics, such as face-wash cosmetics, basic cosmetics, color cosmetics, hair cosmetics, functional cosmetics, etc. Herein, as the additive constituting the cosmetic, a known additive for preparing the cosmetic may be used. A detailed description of the cosmetic additive will be omitted.

The solvent in the examples is ethanol or cosmetics, but is not limited thereto, and any solvent that can be added to food or cosmetics may be used without limitation.

FIG. 2 is a flowchart for explaining the steps of preparing a Dahlia pinnata extract (S 100) according to an embodiment of the present invention.

Referring to FIG. 2, the step of preparing Dahlia pinnata extract may include preparing Dahlia pinnata petals (S 110), adding ethanol to the Dahlia pinnata petals and stirring them to extract Dahlia pinnata extract (S 120), and filtering the Dahlia pinnata extract (S 130).

The step of preparing Dahlia pinnata petals (S 110) may include removing foreign substances and preparing the washed Dahlia pinnata petals. For example, 20g of Dahlia pinnata petals may be prepared by removing foreign substances and washing. However, this is only an example, and the amount of Dahlia pinnata petals may be changed according to the capacity of Dahlia pinnata composition to be prepared.

In the step of extracting Dahlia pinnata extract (S120), ethanol may be added to the prepared Dahlia pinnata petals and stirred to extract the Dahlia pinnata extract comprising the antioxidant component. Herein, Dahlia pinnata extract may be extracted by adding 5 to 20ml of ethanol per 1g of Dahlia pinnata petals. As a more preferred example, Dahlia pinnata extract may be extracted by adding 10ml of ethanol per 1g of Dahlia pinnata petals. For example, 20ml of ethanol may be added to 20g of Dahlia pinnata petals, and Dahlia pinnata extract may be extracted by stirring the Dahlia pinnata petals at a speed of 30rpm at room temperature for 5 hours.

If Dahlia pinnata flowers are extracted by distillation or pressing, their constituents may be destroyed. Therefore, it is preferable in the step of extracting Dahlia pinnata extract, ethanol is added to Dahlia pinnata petals for extraction, and more specifically, ethanol is added to the Dahlia pinnata petals to leach the constituents, and then, ethanol as the solvent is removed to extract Dahlia pinnata flower, ensuring that the components are not destroyed. In addition to ethanol, the Dahlia pinnata extract may be extracted with volatile organic solvents such as petroleum ether, benzine, and chloroform, but the solvent is not suitable for preparing a Dahlia pinnata composition used in food or cosmetic due to toxicity of the remaining organic solvent.

In step of filtering the Dahlia pinnata extract (S130), Dahlia pinnata extract may be filtered with a filter and stored at a temperature of about 4°C. For example, Dahlia pinnata extract may be filtered with a syringe filter (HM) having a size of 25mm and stored in a refrigerator at about 4°C.

Herein, the syringe filter is a disposable filter used to filter suspended matter in a liquid sample. The Syringe filter includes a membrane filter that may separate mixtures by separating and passing certain components through.

The syringe filter may be attached to the tip of a syringe to apply a constant pressure, and then filter through the micropores of the membrane filter. However, the syringe filter is only an example, and may be changed to various filters capable of filtering the Dahlia pinnata extract.

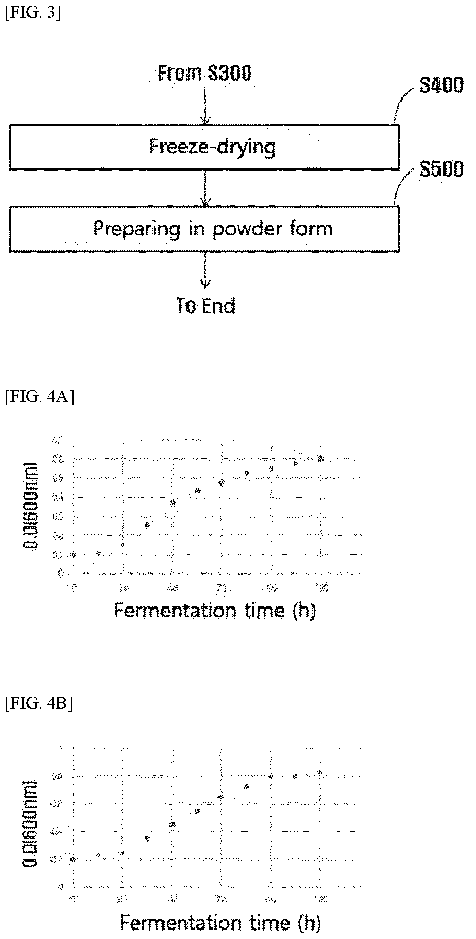

Such a Dahlia pinnata composition may be provided in a powder form. Hereinafter, referring to FIG. 3, a method for preparing Dahlia pinnata composition in a powder form will be described in detail.

FIG. 3 is a flowchart of a method for preparing a Dahlia pinnata composition in a powder form, according to an embodiment of the present invention.

Referring to FIG. 3, the method for preparing Dahlia pinnata composition in a powder form may include: freeze-drying Dahlia pinnata composition prepared by the above-described method (S400) and preparing the freeze-dried Dahlia pinnata composition in a powder form (S500).

In the step of freeze-drying Dahlia pinnata composition (S400), the Dahlia pinnata composition may be put into a refrigerator using liquid nitrogen as a refrigerant and rapidly frozen at a temperature of -70°C or less. The Dahlia pinnata composition may be vacuum dried to reduce the moisture content.

In the step of preparing the freeze-dried Dahlia pinnata composition in a powder form (S500), the freeze-dried Dahlia pinnata composition may be put into an ultra-low temperature freeze mill for freeze-pulverizing. Liquid nitrogen is injected into the cryogenic freeze mill to reduce heat generated during the grinding process with grinding. Pulverized Dahlia pinnata composition has a powder form. The Dahlia pinnata extract in a powder form may be used by being adding to food or cosmetics.

It was found that the Dahlia pinnata composition in liquid or powder form comprises a phenolic compound having an antioxidant efficacy, and also has a high antioxidant efficacy in the antioxidant measurement. Since Dahlia pinnata flowers have various colors, they may be used as natural pigments in foods and cosmetics, and have antioxidant efficacy, so they may have anti-aging effects. In addition, the Dahlia pinnata composition comprising the Dahlia pinnata fermented extract may represent skin barrier strengthening and anti-inflammatory effects by increasing the expression level of the skin keratinocyte protein and moisturizing factors and suppressing the generation of inflammatory factors.

## Examples

Hereinafter, the present invention will be described in more detail through examples. However, these examples show some experimental methods and compositions for illustrative purposes of the present invention, and the scope of the present invention is not limited to these Examples.

### Preparation Example 1: Preparation of Dahlia Pinnata Fermented Extract

### Preparation of Dahlia pinnata extract and selection of fermentation strains

200ml of ethanol was added to 20g of the Dahlia pinnata petals, the Dahlia pinnata extract was extracted by stirring at 30rpm at room temperature for 5 hours, and the filtrate was filtered through a 25mm syringe filter to prepare the Dahlia pinnata extract.

In order to discover strains suitable for fermenting the Dahlia pinnata extract, 1,501 strains of microorganisms were collected from various sources, and 36 strains of Bacillus genus having high amylase and protease activities were selected. Table 1 shows the types and sources of the selected 36 Bacillus genus strains.

[Table 1]

| Sample number | Kind of strains | Source | Sample number | Kind of strains | Source |
|---|---|---|---|---|---|
| 1 | MSP-7 | Moseulpo Port | 19 | NWFO-14-2 | Nockwon farm |
| 2 | CTC 1-4-1 | Clam fermented food | 20 | NWFY-11-2 | Nockwon farm |
| 3 | CTC 2-5-1 | Clam fermented food | 21 | NWFY-36 | Nockwon farm |
| 4 | DJ-3-61 | Daej eo Ecological Park | 22 | NWFY-41 | Nockwon farm |
| 5 | DPP-15 | Daepo port | 23 | OGH-18 | Salted cutlassfish of olleh market |

(continued)

| Sample number | Kind of strains | Source | Sample number | Kind of strains | Source |
|---|---|---|---|---|---|
| 6 | DPS-3-1 | Soil near Daepo port | 24 | OGH-19 | Salted cutlassfish of olleh market |
| 7 | EF45011 | Ocher oak grass | 25 | OGH-24 | Salted cutlassfish of olle market |
| 8 | GC-3-13 | Across Amnam Park near Gamcheon Port of Pusan | 26 | OGH-8 | Salted cutlassfish of olleh market |
| 9 | GIPS-2-2-3 | Geumil Beach | 27 | YS-DS 3C | Yeosu Dolsan Park |
| 10 | JSP-1-11-1 | Soybean fermented food | 28 | YS-DS 4C | Yeosu Dolsan Park |
| 11 | JSP-1-4-1 | Soybean fermented food | 29 | YS-YR 5A | Yeosu Yeondeungcheon Namsan Bridge |
| 12 | JSP-1-6-1 | Soybean fermented food | 30 | R450301 | Rock salt |
| 13 | JSP-2-9-2 | Soybean fermented food | 31 | EF45023 | Gangwon Charcoal |
| 14 | KOS-3-1-1 | Korean observatory soil | 32 | NWFY-19 | Nockwon farm |
| 15 | KOS-3-2-2 | Korean observatory soil | 33 | R450403 | Rock salt |
| 16 | MSW-3-1-1 | Mogihama Beach | 34 | OGH-20 | Salted cutlassfish of olleh market |
| 17 | MY-DS 3C | Korean observatory soil | 35 | GS-BC 3A | Pickled large-eyed herring |
| 18 | NWFO-14-1 | Nockwon farm | 36 | R450401 | Rock salt |

For these strains, it was confirmed that growth was observed in Dahlia pinnata extract-containing medium, and as a result, only two strains of *Bacillus licheniformis* R450403 and *Bacillus amyloliquefaciens* CTC 2-5-1 out of 36 strains were possible to grow in Dahlia pinnata extract medium, and all of the remaining 34 strains did not grow.

FIG. 4A is a growth curve in *Bacillus licheniformis* R450403, FIG. 4B is a growth curve in *Bacillus amyloliquefaciens* CTC 2-5-1, and both are shown as the shape of the sigmoidal curve.

### Preparation of Dahlia Pinnata Fermented Extract

Dahlia pinnata extract was fermented with a selected strain to prepare Dahlia pinnata fermented extract.

The medium used for fermentation (0.4% nutrient broth) was composed of 10% by weight of Dahlia pinnata extract, the medium was inoculated with *Bacillus licheniformis* R450403 or *Bacillus amyloliquefaciens* CTC 2-5-1 followed by incubation at 37°C for 5 days for fermentation, the supernatant was collected to prepare Dahlia pinnata fermented extract.

### Experimental Example 1: Analysis of DPPH radical scavenging ability

DPPH radical scavenging activity was analyzed for the Dahlia pinnata fermented extract obtained in Preparation Example 1.

Ethanol was added to the Dahlia pinnata fermented extract as a solvent to prepare a Dahlia pinnata composition as shown in Table 2 below.

[Table 2]

| Content | Concentration of Dahlia pinnata fermented extract (mg/ml) | Kind of strains |
|---|---|---|
| Example 1 | 3.45 | *Bacillus licheniformis* R450403 |
| Example 2 | 7.13 | |
| Example 3 | 14.25 | |
| Example 4 | 3.38 | *Bacillus amyloliquefaciens* CTC 2-5-1 |
| Example 5 | 6.75 | |
| Example 6 | 13.60 | |

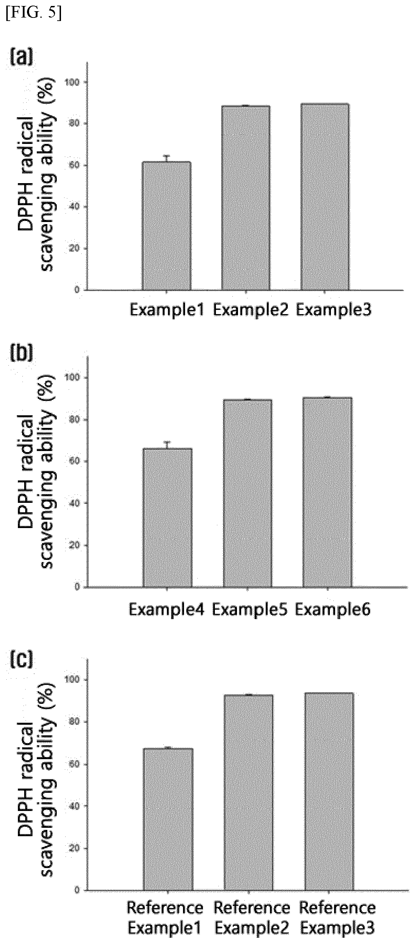

Specifically, 150μl of 0.2mM DPPH solution dissolved in 100% ethanol was added to 50μl of a sample, mixed therewith, and reacted for 30 minutes by blocking light at room temperature. Then, the absorbance was measured at 517nm using a microplate reader (Synergy™ HTX Multi-Mode Microplate Reader, Biotek) to yield the DPPH radical scavenging ability, and the results were shown in FIG. 5. The DPPH radical scavenging ability was calculated according to Equation 1 below.

[Equation 1]

$$\text{DPPH radical scavenging ability}(\%) = [1-(A_{sample}-A_{blank})/(B_{control}-B_{blank})] \times 100$$

wherein $A_{sample}$ is the absorbance value of the reaction between each sample and the DPPH solution, $A_{blank}$ is the absorbance value of the sample color, $B_{control}$ is the absorbance value of the reaction between ethanol as the solvent of DPPH, and the DPPH solution, and $B_{blank}$ is the absorbance of the color of the solvent itself.

Meanwhile, as a positive control, ethanol was added to ascorbic acid (L-Ascorbic acid, Sigma Aldrich, 50-81-7, USA) so that a concentration of ascorbic acid contained in ethanol was formulated to be 25μg/ml (Reference Example 1), 50μg /ml (Reference Example 2), and 100μg/ml (Reference Example 3). The ascorbic acid is an organic compound with antioxidant properties. The ascorbic acid is a type of vitamin C and has a lactone structure. The ascorbic acid is an optically active compound and is widely used as an antioxidant in food additives. For Reference Examples 1 to 3, the DPPH radical scavenging ability was calculated in the same manner as in Examples, and the results are shown in FIG. 5 together.

FIG. 5A is a graph of the DPPH radical scavenging ability of Examples 1 to 3, FIG. 5B is a graph of the measured value of the DPPH radical scavenging ability of Examples 4 to 6, and FIG. 5C is a graph of DPPH radical scavenging capacity measurements of Reference Examples 1 to 3.

From the graph of FIG. 5, it was confirmed that the Dahlia pinnata fermented extract which was fermented using the fermentation strain of the present invention can exhibit antioxidant activity similar to or superior to ascorbic acid.

**Experimental Example 2: Analysis of ABTS radical scavenging ability**

The ABTS radical scavenging activity was analyzed for the same Dahlia pinnata composition as used in Experimental Example 1.

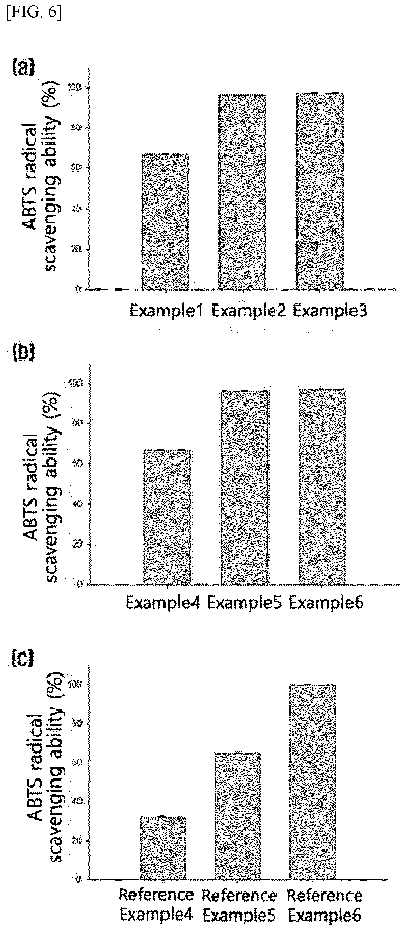

Specifically, the ABTS reaction solution was used by diluting the solution in the range of absorbance value of 0.68 to 0.72 after dark reaction of 7mM ABTS solution and 2.45mM potassium persulfate ($K_2S_2O_8$) which are dissolved in water with a volume ratio of 1:1 for 24 hours. In order to measure the ABTS radical scavenging ability, 100μl of an ABTS solution was added to 100μl of a sample, with mixing and reacting the sample at room temperature for 5 minutes, and then absorbance was measured at 734nm using a microplate reader (Synergy™ HTX Multi-Mode Microplate Reader, Biotek) to yield ABTS radical scavenging ability, and the results are shown in FIG. 6. The ABTS radical scavenging ability was calculated according to Equation 2 below.

[Equation 2]

$$\text{ABTS radical scavenging ability}(\%) = [1-(A_{sample}-A_{blank})/(B_{control}-B_{blank})] \times 100$$

wherein $A_{sample}$ is the absorbance value of the reaction between each sample and the ABTS solution, $A_{blank}$ is the absorbance value of the sample color, $B_{control}$ is the absorbance value of the reaction between water as the solvent of ABTS, and the ABTS solution, and $B_{blank}$ is the absorbance of the color of the solvent itself.

Meanwhile, as a control, ethanol was added to ascorbic acid (L-Ascorbic acid, Sigma Aldirich, 50-81-7, USA) so that the concentration of ascorbic acid contained in ethanol was formulated to be 5μg/ml (Reference Example 4), 10μg /ml (Reference Example 5) and 20μg/ml (Reference Example 6). For Reference Examples 4 to 6, the ABTS radical scavenging ability was calculated in the same manner as in Examples, and the results are shown in FIG. 6 together.

FIG. 6A is a graph of the ABTS radical scavenging ability of Examples 1 to 3, FIG. 6B is a graph of the measured value of the ABTS radical scavenging ability of examples 4 to 6, and FIG. 6C is a graph of the measured value of the ABTS radical scavenging ability of Reference Examples 4 to 6.

Like a result of Experimental Example 3, it was confirmed that the Dahlia pinnata fermented extract which was fermented using the fermentation strain of the present invention can exhibit antioxidant activity similar to or superior to ascorbic acid.

## Experimental Example 3: Determination of total phenolic compounds content

The total phenolic compound (polyphenol) content was measured for the Dahlia pinnata compositions of Examples 3 and 6 using a Folin-ciocalteu analysis method. 20μl of a 1N Folin-ciocalteu reagent was mixed with 80μl of a Dahlia pinnata composition, and reacted at room temperature for 5 minutes. Then, 100μl of a 2% sodium carbonate ($Na_2CO_3$) solution was dispensed and reacted for 10 minutes. Thereafter, the absorbance was measured at 750nm using a microplate reader (Synergy™ HTX Multi-Mode Microplate Reader, Biotek). Gallic acid (Sigma Aldrich, USA) was used as a standard material.

Table 3 shows the content of the total phenolic compounds for the Dahlia pinnata fermented extract.

[Table 3]

| | Concentration of Dahlia pinnata fermented extract (mg/ml) | Content of phenolic compound (% by weight) | Concentration of phenolic compound (mg/ml) | Content Of phenolic compound (μg/100 mg) |
|---|---|---|---|---|
| Example 3 | 14.25 | 61.22 | 8.7238 | 872.38 |
| Example 6 | 13.60 | 64.27 | 8.7407 | 874.07 |

It can be seen from Table 3 that the Dahlia pinnata composition according to the present invention comprises a large amount of a phenolic compound having an antioxidant component, and thus has an excellent antioxidant function. Particularly, this corresponds to a 2.6-fold increase in the content of total phenolic compound, compared to the Dahlia pinnata composition comprising the Dahlia pinnata extract (non-fermented product) which was analyzed as having the content of the total phenolic compound of about 331.91μg/mg. Therefore, it can be seen that when the Dahlia pinnata composition according to the present invention is used for food or cosmetics, a significantly excellent antioxidant effect may be achieved even with a small amount.

## Experimental Example 4: Antioxidant efficacy analysis

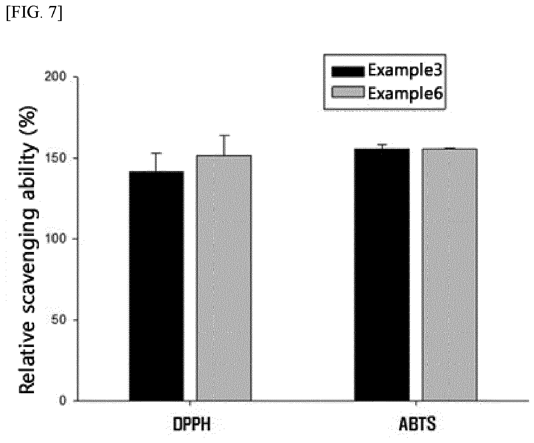

For each of the Dahlia pinnata compositions of Examples 3 and 6, a control containing unfermented Dahlia pinnata extract was prepared, and their DPPH radical scavenging ability and ABTS radical scavenging ability were measured, and thereby, the degree of increase in antioxidant efficacy of Examples 3 and 6 compared to the control group was respectively evaluated. The evaluation results of their relative scavenging activity (%) were shown in FIG. 7. It was confirmed from FIG. 7 that in the case of a fermented product using *Bacillus licheniformis*, the DPPH radical scavenging ability was increased by about 1.4 times, and the ABTS radical scavenging ability was increased by about 1.5 times. Also, it was confirmed that in the case of the fermented product using *Bacillus amyloliquefaciens*, both the DPPH radical scavenging ability and the ABTS radical scavenging ability were increased by about 1.5 times.

**Experimental Example 5: Analysis of skin barrier strengthening efficacy**

Experimental method

For the fermented extract obtained in Preparation Example 1, the skin barrier strengthening efficacy was tested by measuring the level of the relevant gene using a real time quantitative polymeric chain reaction (RT-qPCR).

The human keratinocyte line (HaCaT) was cultured in a $CO_2$ incubator at 37°C under 5% $CO_2$ conditions. In this case, as the culture medium, DMEM (Dulbecco's modified Eagle's Medium) medium containing 10% fetal bovine serum (FBS) and 1% penicillin/streptomycin (P/S) antibiotic was used. A maximum of 80% confluency was maintained during cell culture, and then the cells were maintained by performing subcultivation, and cultured at a condition of $4.5 \times 10^5$ cells/well (cells/well).

The cultured human keratinocyte line was treated with the Dahlia pinnata fermented extract samples of Preparation Example 1 in amounts of 0mg/ml, 2mg/ml, 4mg/ml, and 6mg/ml, respectively, and cultured for 3 hours. The Dahlia pinnata fermented extract was used in the experiment by adding ethanol as a solvent, drying the Dahlia pinnata composition in a drying oven, redispersing the dried Dahlia pinnata composition, and then performing filtration.

Cultured cells were harvested, and intracellular RNA was isolated using a RNAiso Plus (Takara Biochemical Inc., Japan). Using the isolated RNA, cDNA was synthesized with a SuPrimeScript RT premix (GeNet Bio, Daejeon, Korea) and RT-qPCR was performed using TaqMan probe to analyze the relative expression pattern of skin barrier strengthening factors. As skin barrier strengthening factors, filaggrin and keratin 1, which are moisturizing factors involved in corneocyte formation, were used. The expression level was corrected based on the mRNA expression level of glyceraldehyde 3-phosphate dehydrogenase (GAPDH).

5-1. Analysis of the expression level of filaggrin

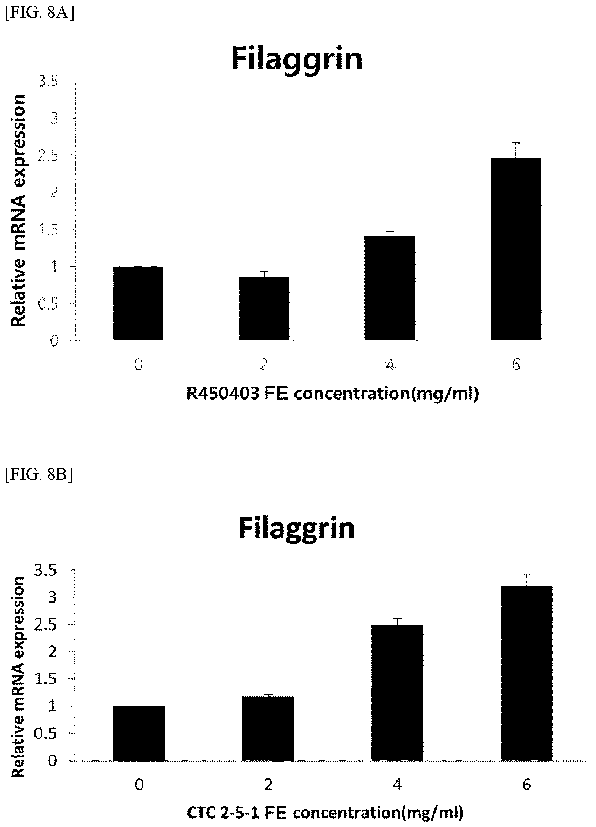

FIGS. 8A and 8B show the results of measuring the expression level of filaggrin after treating the Dahlia pinnata fermented extract, which was fermented with R450403 and CTC 2-5-1, respectively. Referring to FIGS. 8A and 8B, when the human keratinocyte line was treated with the Dahlia pinnata fermented extract of the present invention, the results showed a tendency to increase the gene expression level of filaggrin compared to the control group (0mg/ml).

Specifically, referring to FIG. 8A, it was confirmed that when the human keratinocyte line was treated with the Dahlia pinnata fermented extract, which was fermented with *Bacillus licheniformis* R450403, the expression level of filaggrin at concentration condition of 4 and 6mg/ml was higher than that of the control (0mg/ml) by 1.41 times (±0.06) and 2.45 times (±0.22), respectively.

In addition, referring to FIG. 8B, it was confirmed that when the human keratinocyte line was treated with the Dahlia pinnata extract fermented with Bacillus amyloliquefaciens CTC 2-5-1, as concentrations of the Dahlia pinnata fermented extract were increased to 2, 4 and 6mg/ml, the expression level of filaggrin was increased by 1.17 times (±0.04), 2.49 times (±0.11), and 3.20 times (±0.23), respectively, compared to the control group (0mg/ml).

5-2. Analysis of expression level of keratin 1

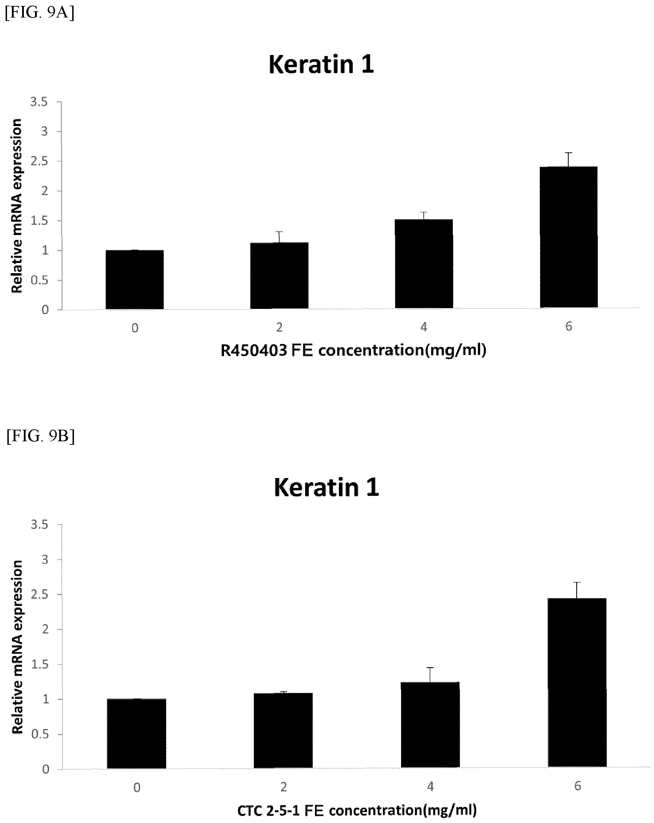

FIGS. 9A and 9B show the results of measuring the expression level of keratin 1 after treating the Dahlia pinnata fermented extract, which was fermented with R450403 and CTC 2-5-1, respectively. Referring to FIGS. 9A and 9B, it was confirmed that when the human keratinocyte line was treated with the Dahlia pinnata fermented extract of the present invention, the gene expression level of keratin 1 was increased, compared to the control group (0mg/ml).

Specifically, referring to FIG. 9A, it was confirmed that when the human keratinocyte line was treated with the Dahlia pinnata extract fermented with *Bacillus licheniformis* R450403, as concentrations of the Dahlia pinnata fermented extract were increased to 2, 4 and 6mg/ml, the expression level of keratin 1 was increased by 1.11 times (±0.19), 1.50 times (±0.12), and 2.38 times (±0.22), respectively, compared to the control group (0mg/ml).

In addition, referring to FIG. 9B, it was confirmed that when the human keratinocyte line was treated with the Dahlia pinnata extract fermented with *Bacillus amyloliquefaciens* CTC 2-5-1, as concentrations of the Dahlia pinnata fermented extract were increased to 2, 4 and 6mg/ml, the expression level of keratin 1 was increased by 1.07 times (±0.02), 1.23 times (±0.21), and 2.42 times (±0.23), respectively, compared to the control group (0mg/ml).

As a result of the skin barrier strengthening efficacy test, it was confirmed that when the human keratinocyte line was treated with the Dahlia pinnata fermented extract of the present invention, the gene expression levels of filaggrin and keratin 1, which act as moisturizing factors as the protein involved in forming stratum corneum, were increased, and from the results, effects in strengthening the skin barrier was confirmed.

**Experimental Example 6: Analysis of anti-inflammatory efficacy**

Experimental method

For the fermented extract obtained in Preparation Example 1, the effect of inhibiting anti-inflammatory-related factor was measured to test the skin soothing effect.

Inflammatory immune cells, RAW 264.7 cells, were cultured in a $CO_2$ incubator at 37°C under 5% $CO_2$ conditions. In this case, as the culture medium, DMEM (Dulbecco's modified Eagle's Medium) medium containing 10% fetal bovine serum (FBS) and 1% penicillin/streptomycin (P/S) antibiotic was used, and RAW 264.7 cells were seeded in a 24-well plate at $2.5 \times 10^5$ cells/well and cultured for 24 hours.

Inflammation was induced by treating the cultured cells with lipopolysaccharide (LPS) at a concentration of 500ng/ml. After 16 hours, the Dahlia pinnata fermented extract samples of Preparation Example 1 were treated in amounts of 0mg/ml, 4mg/ml, 6mg/ml and 8mg/ml, respectively, and cultured for 24 hours in an incubator. The Dahlia pinnata fermented extract was used in the experiment by adding ethanol as a solvent, drying the Dahlia pinnata composition in a drying oven, redispersing the dried Dahlia pinnata composition, and then performing the filtration.

After culturing, the supernatant was collected and centrifuged at 12,000 rpm for 2 minutes, and the production amount of nitric oxide (NO) and interleukin-1 beta (IL-1β) as inflammatory factors was measured.

6-1. Nitric oxide assay (NO assay)

The anti-inflammatory effect was analyzed by measuring the amount of nitric oxide, one of the inflammatory products.

Specifically, 100μL of a Griess reagent containing 1%(w/v) sulfanilamide and 0.1%(w/v) naphthylethylene diamine in a 2.5%(v/v) phosphoric acid solution was mixed with 100μL of the centrifuged supernatant, and reacted for 10 minutes in a 96-well plate.

After the reaction, absorbance was measured at 540nm using an absorbance spectrometer (Synergy™ HTX Multi-Mode Microplate Reader, Biotek), and the concentration of the produced nitrite was quantitatively analyzed using sodium nitrite. For comparison, the concentration of nitrite was quantitatively analyzed for the untreated group that was not performed with a treatment of both the induction of inflammation and the Dahlia pinnata fermented extract.

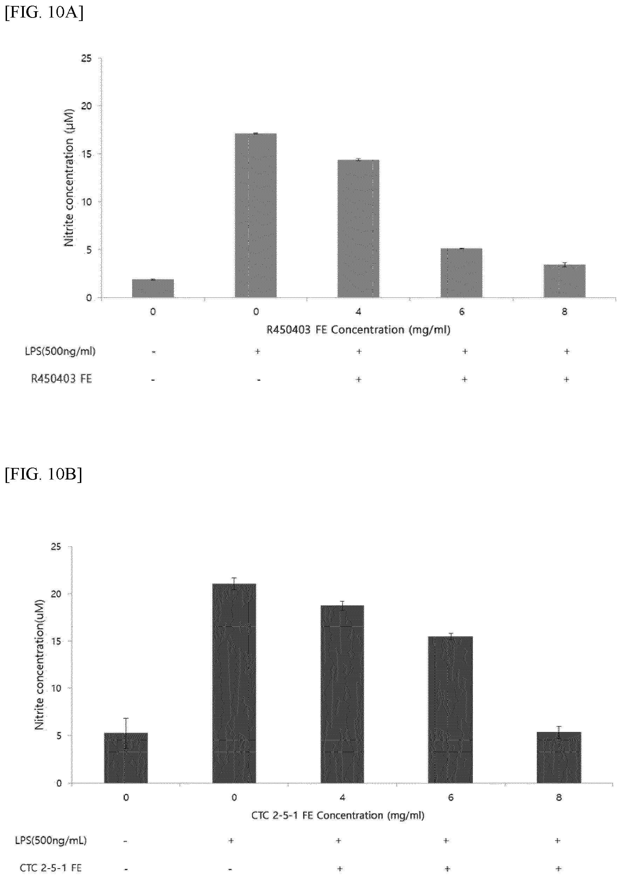

FIGS. 10A and 10B show the results of measuring the concentration of nitrite after treating the Dahlia pinnata fermented extract, which was fermented with R450403 and CTC 2-5-1, respectively. Referring to FIGS. 10A and 10B, it was confirmed that the concentration of nitrite was decreased when the inflammation-induced RAW 264.7 cells were treated with the Dahlia pinnata fermented extract of the present invention. In addition, it was confirmed that the higher the concentration of the extract, the lower the amount of nitrite produced.

Specifically, referring to FIG. 10A, it was confirmed that when the Dahlia pinnata fermented extract, which was fermented with *Bacillus licheniformis* R450403 was treated, the concentration of nitrite was significantly reduced at the extract concentration of 6mg/ml or more, and the nitrite concentration was reduced to the extent that it was almost close to the level of the untreated group at the 8mg/ml condition.

In addition, referring to FIG. 10B, it was confirmed that when the Dahlia pinnata fermented extract, which was fermented with *Amyloliquefaciens* CTC 2-5-1, was treated, the nitrite concentration was reduced to the extent that it was close to the level of the untreated group at the extract concentration of 8 mg/ml.

6-2. Interleukin-1 beta (IL-1β) analysis

The anti-inflammatory effect was analyzed by measuring the concentration of interleukin-1 beta as a cytokine related to inflammatory diseases.

For the centrifuged supernatant, an enzyme-linked immunosorbent assay (ELISA) was performed using an IL-1β ELISA Kit (Duoset, R&D systems), and the amount of interleukin-1 beta was quantified using an absorbance spectrophotometer (Synergy™ HTX Multi-Mode Microplate Reader, Biotek) at 450nm. For comparison, the same experiment was subject to the untreated group that was not performed with a treatment of both the induction of inflammation and the Dahlia pinnata fermented extract.

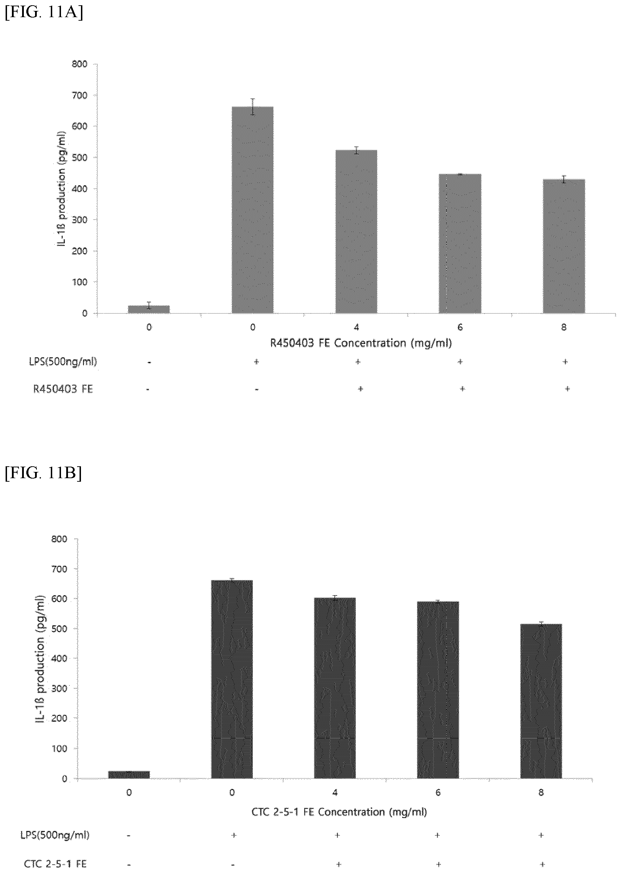

FIGS. 11A and 11B show the results of measuring the amount of interleukin-1 beta after treating the Dahlia pinnata fermented extract, which was fermented with R450403 and CTC 2-5-1, respectively.

Referring to FIGS. 11A and 11B, it could be confirmed that when RAW 264.7 cells are treated with LPS, inflammation is induced and the concentration of interleukin-1 beta sharply increases, and when the Dahlia pinnata fermented extract according to the present invention is treated, the concentration of interleukin-1 beta decreases. In addition, it was confirmed that the production amount of interleukin-1 beta decreased as the concentration of the extract increased, and the tendency of the production amount of interleukin-1 beta of the sample fermented with R450403 to be lower than that of the sample fermented with CTC 2-5-1 was observed.

As a result of the anti-inflammatory efficacy test, when the cells induced in inflammation was treated with the Dahlia pinnata fermented extract of the present invention, the production of inflammatory factors was inhibited. From this, it was confirmed that the Dahlia pinnata fermented extract according to the present invention exhibits excellent skin immunity enhancement and inflammation alleviation effects.

As the specific part of the content of the present invention has been described in detail above, it will be obvious for those of ordinary skill in the art that this specific description is only a preferred embodiment, and the scope of the present invention is not limited thereby. Therefore, it will be said that the substantial scope of the present invention is defined by the appended claims.

**Claims**

1. A cosmetic composition comprising, as an active ingredient, *Dahlia pinnata* fermented extract obtained by inoculating Dahlia pinnata extract with one or more Bacillus sp. strains selected from the group consisting of *Bacillus licheniformis* and *Bacillus amyloliquefaciens*, and fermenting the extract.

2. The cosmetic composition of claim 1,
   **characterized in that** the Bacillus sp. strains are one or more selected from the group consisting of *Bacillus licheniformis* R450403 (Deposit accession number: KCTC14348BP) and *Bacillus amyloliquefaciens* CTC 2-5-1 (Deposit accession number: KCTC14347BP).

3. The cosmetic composition of claim 1,
   **characterized in that** the concentration of the Dahlia pinnata fermented extract is 1 to 20mg/ml.

4. The cosmetic composition of claim 1,
   **characterized in that** the Dahlia pinnata extract is ethanol extract of Dahlia pinnata petals.

5. The cosmetic composition of claim 1,
   **characterized in that** a content of total phenolic compounds is 50 to 80% by weight, based on the Dahlia pinnata fermented extract.

6. The cosmetic composition of claim 1,
   **characterized in that** the Dahlia pinnata fermented extract is improved in one or more of 2,2-diphenyl-1-picrylhydrazyl (DPPH) free radical scavenging activity and 2,2'-azino-bis-3-ethyl benzothiazoline-6-sulphonic acid (ABTS) free radical scavenging ability, compared to the Dahlia pinnata extract.

7. The cosmetic composition of claim 1,
   **characterized in that** the composition has antioxidant and anti-aging activity.

8. The cosmetic composition of claim 1,
   **characterized in that** the composition increases an expression of one or more proteins selected from filaggrin and keratin 1 in skin cells to strengthen a skin barrier.

9. The cosmetic composition of claim 1,
   **characterized in that** the composition exhibits an anti-inflammatory effect by inhibiting a production of one or more inflammatory factors selected from nitric oxide (NO) and interleukin-1β (IL-1β).

10. A method for preparing a Dahlia pinnata composition, comprising the steps of:

    preparing Dahlia pinnata extract,
    inoculating the Dahlia pinnata extract with one or more Bacillus sp. strains selected from the group consisting of *Bacillus licheniformis* and *Bacillus amyloliquefaciens*, and then fermenting the extract to obtain Dahlia pinnata fermented extract, and
    diluting the Dahlia pinnata fermented extract in a solvent to obtain a Dahlia pinnata composition.

11. The method of claim 10,
    **characterized in that** the fermentation is performed for 72 to 144 hours at a temperature of 25 to 45°C.

**12.** The method of claim 10,
**characterized in that** the Bacillus sp. strains are inoculated in an amount of 1 to 10 parts by volume based on 100 parts by volume of the Dahlia pinnata extract.

**13.** The method of claim 10,
**characterized in that** the step of preparing the Dahlia pinnata extract includes:

preparing Dahlia pinnata petals,
adding ethanol to the Dahlia pinnata petals and stirring them to extract Dahlia pinnata extract, and
filtering the Dahlia pinnata extract.

**14.** The method of claim 13,
**characterized in that** the step of extracting the Dahlia pinnata extract includes:
adding 5 to 20ml of ethanol per 1g of the Dahila pinnata petals to extract the Dahlia pinnata extract.

**15.** The method of claim 13,
**characterized in that** the step of filtering the Dahlia pinnata extract includes:
filtering the Dahlia pinnata extract by a sylinge filter.

**16.** The method of claim 10, further comprising

freeze-drying the Dahlia pinnata composition, and
preparing the freeze-dried Dahlia pinnata composition in a powdered form.

**17.** A Dahlia pinnata composition prepared by the method of any one of claims 10 to 16, and comprising the Dahlia pinnata fermented extract.

**Patentansprüche**

**1.** Kosmetische Zusammensetzung, umfassend als Wirkstoff einen fermentierten *Dahlia pinnata*-Extrakt, der durch Inokulieren von Dahlia pinnata-Extrakt mit einem oder mehreren Bacillus sp.-Stämmen, ausgewählt aus der Gruppe, bestehend aus *Bacillus licheniformis* und *Bacillus amyloliquefaciens*, und Fermentieren des Extrakts erhalten wurde.

**2.** Kosmetische Zusammensetzung nach Anspruch 1,
**dadurch gekennzeichnet, dass** die Bacillus sp.-Stämme einer oder mehrere sind, ausgewählt aus der Gruppe bestehend aus *Bacillus licheniformis* R450403 (Hinterlegungsnummer: KCTC14348BP) und *Bacillus amyloliquefaciens* CTC 2-5-1 (Hinterlegungsnummer: KCTC14347BP).

**3.** Kosmetische Zusammensetzung nach Anspruch 1,
**dadurch gekennzeichnet, dass** die Konzentration des fermentierten Dahlia pinnata-Extrakts 1 bis 20 mg/ml beträgt.

**4.** Kosmetische Zusammensetzung nach Anspruch 1,
**dadurch gekennzeichnet, dass** der Dahlia pinnata-Extrakt ein Ethanolextrakt aus Dahlia pinnata-Blütenblättern ist.

**5.** Kosmetische Zusammensetzung nach Anspruch 1,
**dadurch gekennzeichnet, dass** der Gehalt an gesamten phenolischen Verbindungen 50 bis 80 Gew.-%, bezogen auf den fermentierten Dahlia pinnata-Extrakt, beträgt.

**6.** Kosmetische Zusammensetzung nach Anspruch 1,
**dadurch gekennzeichnet, dass** der fermentierte Dahlia pinnata-Extrakt im Vergleich zum Dahlia pinnata-Extrakt in einem oder mehreren von 2,2-Diphenyl-1-picrylhydrazyl (DPPH) freie Radikale Fänger-Aktivität und 2,2'-Azino-bis-3-ethyl-benzothiazolin-6-sulfonsäure (ABTS) freie Radikale Fänger-Aktivität verbessert ist.

**7.** Kosmetische Zusammensetzung nach Anspruch 1,
**dadurch gekennzeichnet, dass** die Zusammensetzung eine antioxidative und Anti-Aging-Aktivität aufweist.

**8.** Kosmetische Zusammensetzung nach Anspruch 1,

**dadurch gekennzeichnet, dass** die Zusammensetzung die Expression eines oder mehrerer Proteine, ausgewählt aus Filaggrin und Keratin 1, in Hautzellen erhöht, um eine Hautbarriere zu stärken.

9.  Kosmetische Zusammensetzung nach Anspruch 1,
    **dadurch gekennzeichnet, dass** die Zusammensetzung eine entzündungshemmende Wirkung aufweist, indem sie die Produktion von einem oder mehreren Entzündungsfaktoren, ausgewählt aus Stickstoffmonoxid (NO) und Interleukin-1β (II,-1β), hemmt.

10. Verfahren zur Herstellung einer Dahlia pinnata-Zusammensetzung, umfassend die folgenden Schritte:

    Herstellung eines Dahlia pinnata-Extrakts,
    Inokulieren des Dahlia pinnata-Extrakts mit einem oder mehreren Bacillus sp.-Stämmen, ausgewählt aus der Gruppe bestehend aus *Bacillus licheniformis* und *Bacillus amyloliquefaciens*, und anschließendes Fermentieren des Extrakts, um einen fermentierten Dahlia pinnata-Extrakt zu erhalten, und
    Verdünnen des fermentierten Dahlia pinnata-Extrakts in einem Lösungsmittel, um eine Dahlia pinnata-Zusammensetzung zu erhalten.

11. Verfahren nach Anspruch 10,
    **dadurch gekennzeichnet, dass** die Fermentation 72 bis 144 Stunden bei einer Temperatur von 25 bis 45°C durchgeführt wird.

12. Verfahren nach Anspruch 10,
    **dadurch gekennzeichnet, dass** die Bacillus sp.-Stämme in einer Menge von 1 bis 10 Volumenteilen, bezogen auf 100 Volumenteile des Dahlia pinnata-Extrakts, inokuliert werden.

13. Verfahren nach Anspruch 10,
    **dadurch gekennzeichnet, dass** der Schritt der Herstellung des Dahlia pinnata-Extrakts umfasst:

    die Herstellung von Dahlia pinnata-Blütenblättern,
    Zugabe von Ethanol zu den Dahlia pinnata-Blütenblättern und Rühren, um den Dahlia pinnata-Extrakt zu extrahieren, und
    Filtern des Dahlia pinnata-Extrakts.

14. Verfahren nach Anspruch 13,
    **dadurch gekennzeichnet, dass** der Schritt des Extrahierens des Dahlia pinnata-Extrakts umfasst:
    Zugabe von 5 bis 20 ml Ethanol pro 1 g der Dahila pinnata-Blütenblätter, um den Dahlia pinnata-Extrakt zu extrahieren.

15. Verfahren nach Anspruch 13,
    **dadurch gekennzeichnet, dass** der Schritt des Filterns des Dahlia pinnata-Extrakts Folgendes umfasst:
    Filtern des Dahlia pinnata-Extrakts durch einen Sylingefilter.

16. Verfahren nach Anspruch 10, das ferner umfasst

    Gefriertrocknen der Dahlia pinnata-Zusammensetzung, und
    Herstellen der gefriergetrockneten Dahlia pinnata-Zusammensetzung in einer pulverisierten Form.

17. Dahlia pinnata-Zusammensetzung, hergestellt durch das Verfahren nach einem der Ansprüche 10 bis 16, und umfassend den fermentierten Dahlia pinnata-Extrakt.


**Revendications**

1.  Composition cosmétique comprenant, en tant que substance active, un extrait fermenté de *Dahlia pinnata* obtenu par inoculation d'une ou plusieurs souches de Bacillus sp. sélectionnées parmi le groupe consistant en *Bacillus licheniformis* et *Bacillus amyloliquefaciens* dans un extrait de Dahlia pinnata, et fermentation de l'extrait.

2.  Composition cosmétique selon la revendication 1,

**caractérisée en ce que** les souches de Bacillus sp. sont une ou plusieurs sélectionnées parmi le groupe consistant en *Bacillus licheniformis* R450403 (déposé sous le numéro d'enregistrement : KCTC14348BP) et *Bacillus amyloliquefaciens* CTC 2-5-1 (déposé sous le numéro d'enregistrement : KCTC14347BP).

3. Composition cosmétique selon la revendication 1,
   **caractérisée en ce que** la concentration de l'extrait fermenté de Dahlia pinnata est de 1 à 20 mg/ml.

4. Composition cosmétique selon la revendication 1,
   **caractérisée en ce que** l'extrait de Dahlia pinnata est de l'extrait éthanolique de pétales de Dahlia pinnata.

5. Composition cosmétique selon la revendication 1,
   **caractérisée en ce qu'**une teneur en composés phénoliques totaux est de 50 à 80 % en poids, sur la base de l'extrait fermenté de Dahlia pinnata.

6. Composition cosmétique selon la revendication 1,
   **caractérisée en ce que** l'extrait fermenté de Dahlia pinnata présente une amélioration d'une ou plusieurs parmi l'activité de piégeage de radicaux libres de 2,2-diphényl-1-picrylhydrazyle (DPPH) et la capacité de piégeage de radicaux libres d'acide 2,2'-azino-bis-3-éthylbenzothiazoline-6-sulphonique (ABTS), par comparaison à l'extrait de Dahlia pinnata.

7. Composition cosmétique selon la revendication 1,
   **caractérisée en ce que** la composition présente une activité antioxydante et anti-vieillissement.

8. Composition cosmétique selon la revendication 1,
   **caractérisée en ce que** la composition accroît une expression d'une ou plusieurs protéines sélectionnées parmi la filaggrine et la kératine 1 dans les cellules cutanées pour renforcer la barrière cutanée.

9. Composition cosmétique selon la revendication 1,
   **caractérisée en ce que** la composition présente un effet anti-inflammatoire en empêchant une production d'un ou plusieurs facteurs inflammatoires sélectionnés parmi l'oxyde nitrique (NO) et l'interleukine-1β (IL-1β).

10. Procédé de préparation d'une composition à base de Dahlia pinnata, comprenant les étapes de :

    préparation d'un extrait de Dahlia pinnata,
    inoculation d'une ou plusieurs souches de Bacillus sp. sélectionnées parmi le groupe consistant en *Bacillus licheniformis* et *Bacillus amyloliquefaciens* dans l'extrait de Dahlia pinnata, puis fermentation de l'extrait pour obtenir un extrait fermenté de Dahlia pinnata, et
    dilution de l'extrait fermenté de Dahlia pinnata dans un solvant pour obtenir une composition à base de Dahlia pinnata.

11. Procédé selon la revendication 10,
    **caractérisé en ce que** la fermentation est réalisée pendant 72 à 144 heures à une température de 25 à 45 °C.

12. Procédé selon la revendication 10,
    **caractérisé en ce que** les souches de Bacillus sp. sont inoculées en une quantité de 1 à 10 parties en volume sur la base de 100 parties en volume de l'extrait de Dahlia pinnata.

13. Procédé selon la revendication 10,
    **caractérisé en ce que** l'étape de préparation de l'extrait de Dahlia pinnata inclut :

    la préparation de pétales de Dahlia pinnata,
    l'ajout d'éthanol aux pétales de Dahlia pinnata et leur agitation pour extraire l'extrait de Dahlia pinnata, et
    le filtrage de l'extrait de Dahlia pinnata.

14. Procédé selon la revendication 13,
    **caractérisé en ce que** l'étape d'extraction de l'extrait de Dahlia pinnata inclut :
    l'ajout de 5 à 20 ml d'éthanol par gramme de pétales de Dahlia pinnata pour extraire l'extrait de Dahlia pinnata.

**15.** Procédé selon la revendication 13,
**caractérisé en ce que** l'étape de filtrage de l'extrait de Dahlia pinnata inclut :
le filtrage de l'extrait de Dahlia pinnata par un filtre à seringue.

**16.** Procédé selon la revendication 10, comprenant en outre

la lyophilisation de la composition à base de Dahlia pinnata, et
la préparation de la composition lyophilisée à base de Dahlia pinnata sous forme pulvérulente.

**17.** Composition à base de Dahlia pinnata préparée par le procédé selon l'une quelconque des revendications 10 à 16, et comprenant l'extrait fermenté de Dahlia pinnata.

[FIG. 1]

```
                    ┌─────────────────┐
                    │      Start      │
                    └─────────────────┘
                             │              ⌒ S100
                             ▼
        ┌────────────────────────────────────────┐
        │     Preparing Dahlia pinnata extract    │
        └────────────────────────────────────────┘
                             │              ⌒ S200
                             ▼
        ┌────────────────────────────────────────┐
        │ Obtaining Dahlia pinnata fermented extract │
        └────────────────────────────────────────┘
                             │              ⌒ S300
                             ▼
        ┌────────────────────────────────────────┐
        │   Obtaining Dahlia pinnata composition  │
        └────────────────────────────────────────┘
                             │
                             ▼
                    ┌─────────────────┐
                    │       End       │
                    └─────────────────┘
```

[FIG. 2]

## S100

```
                                              ⌒ S110
        ┌────────────────────────────────────────┐
        │      Preparing Dahlia pinnata petals    │
        └────────────────────────────────────────┘
                             │              ⌒ S120
                             ▼
        ┌────────────────────────────────────────┐
        │     Extracting Dahlia pinnata extract   │
        └────────────────────────────────────────┘
                             │              ⌒ S130
                             ▼
        ┌────────────────────────────────────────┐
        │      Filtering Dahlia pinnata extract   │
        └────────────────────────────────────────┘
```

[FIG. 3]

[FIG. 4A]

[FIG. 4B]

[FIG. 5]

EP 3 995 129 B1

[FIG. 6]

[a] ABTS radical scavenging ability (%) — Example1, Example2, Example3

[b] ABTS radical scavenging ability (%) — Example4, Example5, Example6

[c] ABTS radical scavenging ability (%) — Reference Example4, Reference Example5, Reference Example6

[FIG. 7]

[FIG. 8A]

[FIG. 8B]

[FIG. 9A]

[FIG. 9B]

[FIG. 10A]

| | | | | | |
|---|---|---|---|---|---|
| LPS(500ng/ml) | - | + | + | + | + |
| R450403 FE | - | - | + | + | + |

[FIG. 10B]

| | | | | | |
|---|---|---|---|---|---|
| LPS(500ng/mL) | - | + | + | + | + |
| CTC 2-5-1 FE | - | - | + | + | + |

[FIG. 11A]

[FIG. 11B]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 2018013754 W

- WO 2019146889 A

**Non-patent literature cited in the description**

- **WELSS T ; BASKETTER DA ; SCHRÖDER KR.** In vitro skin irritation: fact and future. State of the art review of mechanisms and models. *Toxicol In Vitro.*, 2004, vol. 18, 231-43